# EUROPEAN PATENT APPLICATION

(11) **EP 4 516 337 A1**
(43) Date of publication of application: **05.03.2025**
(21) Application number: 22939652.8
(22) Date of filing: 15.07.2022
(51) Int. Cl.: A61M 5/14

(54) **MEDICAL DEVICE AND MEDICAL SYSTEM FOR CONVEYING FLUID**

(30) Priority: 26.04.2022 CN 202210444443
(71) Applicant: Shenzhen Sisensing Co., Ltd., Shenzhen, Guangdong 518110 (CN)
(72) Inventor: XIA, Bin, Shenzhen, Guangdong 518110 (CN); LI, Yunfeng, Shenzhen, Guangdong 518110 (CN); HAN, Mingsong, Shenzhen, Guangdong 518110 (CN); CHEN, Zhi, Shenzhen, Guangdong 518110 (CN); FANG, Junfei, Shenzhen, Guangdong 518110 (CN); LAI, Ming, Shenzhen, Guangdong 518110 (CN)
(74) Representative: Isern Patentes y Marcas S.L.
(86) International application number: PCT/CN2022/105855
(87) International publication number: WO 2023/206824

(57) **Abstract**

The present disclosure provides a medical device (10) and a medical system (1) for fluid conveyance, wherein the medical device (10) includes: a reservoir (11), a first passage (12), a flow feeder (13), a driving mechanism (15), a second passage (14), and a flow-limiting valve (16), wherein the flow-limiting valve (16) has a first state and a second state; when the flow-limiting valve (16) is in the first state, the driving mechanism (15) maintains the flow feeder (13) at a first volume, and the flow feeder (13) receives a predetermined volume of fluid from the reservoir (11) via the first passage (12); when the flow-limiting valve (16) is in the second state, the driving mechanism (15) maintains the flow feeder (13) at a second volume, and the flow feeder (13) provides a predetermined volume of fluid via the second passage (14), the second volume being smaller than the first volume and the predetermined volume being determined by a volume difference between the first volume and the second volume. In this case, the effect of digital quantity control can be achieved by obtaining a predetermined volume of fluid for injection so that the fluid can be delivered with high precision.

## Description

### BACKGROUND OF INVENTION

### Field of Invention

The present disclosure relates generally to the field of medical instruments and more particularly to a medical device and a medical system for fluid conveyance.

### Description of Related Art

For many chronic diseases, corresponding complications often arise, e.g., chronic diabetes causing complications related to blood glucose. In order to delay or reduce the rapid or persistent effects of chronic diseases on patients, techniques for delivering drugs by automatic injection may be employed on the patients. In current drug delivery technology, the portable drug delivery system is widely used, which usually requires subcutaneous implantation of one tube, when the abnormality of a patient's physiological characteristics reaches early warning, the patient can input the dose of the injected drug through the controller and connect the drug pump filled with medical solution to the previously reserved tube for drug delivery.

However, with the above-mentioned conventional drug delivery techniques, there is still a great difficulty in accurate delivery. If the delivery quantity is too small or too large, there may be a delay in the response time of the drug or an error in the quantity of the drug used, thus resulting in poor treatment effect or medication error. Accordingly, there is an urgent need for a medical device that conveys fluids with high precision.

### SUMMARY OF INVENTION

The disclosure is accomplished in view of the state of the prior art, and the object of the disclosure is to provide a medical device and a medical system capable of conveying fluids with high precision.

For this purpose, the first aspect of the present disclosure provides a medical device for fluid conveyance, comprising: a reservoir for containing a fluid, a first passage in communication with the reservoir, a flow feeder for receiving the fluid from the reservoir via the first passage and providing the fluid, a driving mechanism for controlling a volume change of the flow feeder, a second passage in communication with the flow feeder, receiving the fluid from the flow feeder and percutaneously accessing a body, and a flow-limiting valve for cooperating with the driving mechanism and controlling an opening and closing of the first passage and the opening and closing of the second passage, wherein the flow-limiting valve has a first state and a second state, the driving mechanism maintains the flow feeder at a first volume when the flow-limiting valve is in the first state, and the flow feeder receives a predetermined volume of fluid from the reservoir via the first passage; the driving mechanism maintains the flow feeder at a second volume when the flow-limiting valve is in the second state, and the flow feeder provides the predetermined volume of fluid via the second passage, the second volume is smaller than the first volume and the predetermined volume is determined by a volume difference between the first volume and the second volume.

In this case, it can be convenient for the patient to carry a large dose of the fluid for injection in case of need for treatment through storing the fluid in the reservoir of the medical device; by means of the cooperation between the flow feeder, the driving mechanism, and the flow-limiting valve, a predetermined volume of fluid is obtained from the reservoir via the first passage, i.e., the difference value between the first volume and the second volume of the flow feeder, the predetermined volume of fluid is infused into the body via the second passage, whereby it is able to obtain a predetermined volume of fluid for transfusion via the medical device when the patient needs the injection of fluid so as to achieve the effect of digital quantity control, resulting in improving the accuracy of the transfusion.

According to the medical device to which the present disclosure relates, optionally, when the flow-limiting valve is in the first state, the flow-limiting valve opens the first passage and closes the second passage; when the flow-limiting valve is in the second state, the flow-limiting valve closes the first passage and opens the second passage. In this case, when the first passage is opened and the second passage is closed, the flow feeder communicating with the first passage can change its volume to a first volume driven by the driving mechanism so as to form a negative pressure and can obtain fluid from the reservoir communicating with the first passage via the first passage; when the first passage is closed and the second passage is open, the flow feeder communicating with the second passage can be restored to its original volume, i.e., the second volume, and can infuse the fluid into the body via the second passage.

According to the medical device to which the present disclosure relates, optionally, the volume of the reservoir is variable, and when the reservoir contains the fluid, the pressure intensity of the fluid of the reservoir is maintained within a predetermined range, with the pressure intensity of the fluid of the reservoir being greater than the pressure intensity of the fluid of the flow feeder. In this case, the fluid is stored in the reservoir and maintained within a predetermined pressure intensity range, enabling the fluid to flow through the first passage into the flow feeder more easily, and serving to prevent fluid backflow or the occurrence of the blood entry resulting in a contaminated fluid.

According to the medical device to which the present disclosure relates, optionally, the reservoir has a sealable supply port. In this case, the reservoir can be periodically replenished with fluid through the supply port; the sealable supply port can maintain the pressure intensity inside the reservoir or reduce the situation of air entering or contamination incurred by the external environment when the reservoir is replenished with the fluid.

According to the medical device to which the present disclosure relates, optionally, the flow-limiting valve includes a first valve controlling the opening and closing of the first passage and a second valve controlling the opening and closing of the second passage, wherein the second valve opens the second passage if the first valve closes the first passage, and the second valve closes the second passage if the first valve opens the first passage. In this case, when the first valve opens the first passage and the second valve closes the second passage, the flow feeder in communication with the first passage can be driven by the driving mechanism to change its volume to the first volume so as to form a negative pressure and can obtain fluids from the reservoir in communication with the first passage via the first passage; when the first valve closes the first passage and the second valve opens the second passage, the flow feeder in communication with the second passage can be restored to the original volume, i.e., a second volume, and can infuse the fluid into the body via the second passage; therefore, with continuously changing the first volume and the second volume, the flow feeder can continuously obtain a predetermined volume of the fluid and inject the same into the body via the second passage.

According to the medical device to which the present disclosure relates, optionally, the driving mechanism includes a first driving mechanism and a second driving mechanism, wherein the first driving mechanism controls the flow-limiting valve to open and close the first passage or the second passage, and the second driving mechanism controls the volume change of the flow feeder to switch the volume of the flow feeder between the first volume and the second volume. In this case, through the cooperation of the first driving mechanism and the second driving mechanism, the first driving mechanism drives the flow-limiting valve to be in a first state, i.e., when the first valve opens the first passage and the second valve closes the second passage, the second driving mechanism can control the volume of the flow feeder to be maintained at the first volume, whereby the flow feeder can form a negative pressure and obtain fluid from the reservoir via the first passage; when the fluid fills the first volume and the first driving mechanism drives the flow-limiting valve to be in the second state, i.e., when the first valve closes the first passage and the second valve opens the second passage, the second driving mechanism is capable of controlling the flow feeder volume to be maintained at the second volume, whereby the flow feeder is capable of infusing a predetermined volume of fluid into the body.

According to the medical device to which the present disclosure relates, optionally, when the first driving mechanism keeps the flow-limiting valve in the first state, the flow-limiting valve opens the first passage and closes the second passage, and the second driving mechanism controls the flow feeder to maintain the first volume and receive the predetermined volume of fluid from the reservoir via the first passage; when the first driving mechanism keeps the flow-limiting valve in the second state, the flow-limiting valve closes the first passage and opens the second passage, and the second driving mechanism controls the flow feeder to maintain the second volume and provides the predetermined volume of fluid via the second passage. In this case, the flow-limiting valve is controlled to switch between the first state and the second state by the first driving mechanism, and the flow feeder is controlled to switch between the first volume and the second volume by cooperating with the second driving mechanism, whereby a predetermined volume of fluid can be continuously obtained and infused into the body, achieving quantitative or digital control, improving the control accuracy of the transfusion.

According to the medical device to which the present disclosure relates, optionally, the flow-limiting valve is of a columnar body and has a first through hole and a second through hole; the first driving mechanism includes a first driver, and a first connector driven by the first driver and connected to the flow-limiting valve; if the first driver drives the first connector so as to rotate the flow-limiting valve to be in the first state, the first through hole is in communication with the first passage, and the second through hole is not in communication with the second passage; if the first driver drives the first connector so as to rotate the flow-limiting valve to be in the second state, the first through hole is not in communication with the first passage, and the second through hole is in communication with the second passage. In this case, via the flow-limiting valve which is of the columnar body, when driven by the first driving mechanism, the first through hole can be in communication with the first passage and the second through hole can be not in communication with the second passage, or the first through hole can be not in communication with the first passage and the second through hole can be in communication with the second passage; therefore, the flow-limiting valve which is of the columnar body can control the flow or non-flow of the fluid via the first passage or the second passage, while the inconvenience of designing multiple valves to control multiple passages can be reduced; in addition, the first driver can provide and transmit power to the flow-limiting valve through the first connector, thereby controlling the flow-limiting valve to switch between the first state and the second state.

According to the medical device to which the present disclosure relates, optionally, the first through hole has a first axis and the second through hole has a second axis, the first axis being orthogonal to the second axis. **In** this case, by rotating the flow-limiting valve which is of the columnar body, for example by degrees clockwise or counterclockwise, the first through hole can be brought into communication with the first passage and the second through hole can be brought out of communication with the second passage, or the first through hole can be brought out of communication with the first passage and the second through hole can be brought into communication with the second passage, , and therefore, it is able to achieve to the effect of controlling the opening and closing of the two passages at the same time by one power.

According to the medical device to which the present disclosure relates, optionally, one side of the reservoir is open and the opening is sealed by a first piston, and the other side of the reservoir is in communication with the first passage. **In** this case, it is able to push the fluid out of the first passage through the piston and maintain a predetermined pressure intensity when the fluid is contained in the reservoir so as to prevent a backflow of the fluid or the blood back flowing and contaminating the fluid in the reservoir.

According to the medical device to which the present disclosure relates, optionally, the first piston is connected to a resilient component, the resilient component being maintained in a compressed state. In this case, the resilient component maintained in the compressed state can drive the first piston to displace so as to maintain the fluid contained in the reservoir at a predetermined pressure intensity, preventing the fluid backflow or blood from back flowing and contaminating the fluid in the reservoir.

According to the medical device to which the present disclosure relates, optionally, the second driving mechanism includes a second driver, and a second connector driven by the second driver and connected to the flow feeder; if the second driver drives the second connector to change the volume of the flow feeder to maintain the first volume, the fluid flows from the reservoir to the flow feeder via the first passage, and if the second driver drives the second connector to change the volume of the flow feeder to maintain the second volume, the fluid flows from the flow feeder to the body via the second passage. In this case, the second driver provides power to be transmitted to the flow feeder via the second connector to change the volume of the flow feeder, thereby enabling a predetermined volume of fluid to be obtained from the reservoir and infused into the body in cooperation with the first driving mechanism and the flow-limiting valve.

According to the medical device to which the present disclosure relates, optionally, one side of the flow feeder is open and the opening is sealed by a second piston, and the other side of the flow feeder is in communication with the second passage. In this case, the volume of the flow feeder can be changed by the displacement of the second piston and a negative pressure can be create at the first volume and a predetermined volume of the fluid can be obtained, the fluid is infused into the body through the second passage when a positive pressure is returned at the second volume .

According to the medical device to which the present disclosure relates, optionally, the second piston is connected to the second connector and driven by the second driver. In this case, the second driver provides power to be transmitted to the second piston via the second connector; therefore, the volume of the flow feeder can be changed by the displacement of the second piston.

According to the medical device to which the present disclosure relates, optionally, the first driver or the second driver is one of a shape memory alloy, a piezoelectric motor, and a servo motor, the first connector is one of a torsion spring and a gear, and the second connector is one of a spring and a connecting rod. In this case, the shape memory alloy is heated to a predetermined range by powering on so as to obtain the power generated by the deformation of the shape memory alloy so that it is able to drive the first connector and the flow-limiting valve; when the heating is stopped, it can recover the force so that the first connector and the flow-limiting valve can be driven reversely, which has the advantages of power saving, accurate control and fast response; a piezoelectric motor or a servo motor drives the first connector and the flow-limiting valve, having the advantages of accurate control and fast response; in addition, the first connector constituted by a torsion spring or a gear can rotate and revolve the flow-limiting valve under power drive, thereby opening and closing the first passage or the second passage; in addition, the second connector constituted by a spring or a push rod can reciprocally displace the second piston under power drive, thereby changing the volume of the flow feeder.

The second aspect of the present disclosure provides a medical system including: the medical device according to any of the first aspect of the present disclosure.

According to the medical system to which the second aspect of the present disclosure relates, optionally, the medical system further includes an external controller that controls the medical device and a sensing monitor communicatively connected to the external controller, wherein the sensing monitor obtains data of a physiological parameters of a user and sends the data of the physiological parameters of the user to the external controller, the external controller controlling the medical device to deliver fluids based on the data of the physiological parameters of the user. In this case, the medical system can accurately provide transfusion therapy to the patient; in addition, the transfusion can be controlled by the external controller and the physiological parameters of the patient can be monitored by the sensing monitor, thereby enabling the medical system to provide transfusion therapy to the patient more accurately and automatically.

According to the present disclosure, it is able to provide a medical device and a medical system for conveying fluids with high precision. Wherein, the medical device controls the conveyance of the fluid by a digital quantity (i.e., a predetermined volume of fluid) so that it is featured with high precision of fluid conveyance compared to the prior art.

### BRIEF DESCRIPTION OF THE DRAWINGS

Fig. 1 is a schematic diagram illustrating an application scenario of an embodiment of a medical system according to an example of the present disclosure.
Fig. 2 is an overall schematic diagram showing a medical device in a medical system according to an example of Fig. 1 of the present disclosure.
Fig. 3 is a schematic diagram showing the structure of a medical device according to an example of the present disclosure.
Fig. 4 is a schematic diagram showing the structure of a reservoir of a medical device according to an example of the present disclosure.
Fig. 5a is a schematic diagram illustrating the flow direction of the fluid in a medical device when a flow-limiting valve is in a first state and a flow feeder is at a first volume according to an example of the present disclosure.
Fig. 5b is a schematic diagram illustrating the flow direction of the fluid in a medical device when a flow-limiting valve is in a second state and a flow feeder is at a second volume according to an example of the present disclosure.
Fig. 6a is a cross-sectional diagram showing a flow-limiting valve (in a first state) of a medical device being connected to a first passage and a second passage according to an example of the present disclosure.
Fig. 6b is a cross-sectional diagram showing a flow-limiting valve (in a second state) of a medical device being connected to a first passage and a second passage according to an example of the present disclosure.
Fig. 7 is a schematic diagram illustrating the process of the rotation of a flow-limiting valve of a medical device from a first state to a second state according to an example of the present disclosure.
Fig. 8 is a schematic diagram showing the structure of a flow feeder of a medical device according to an example of the present disclosure.
Fig. 9 is a schematic diagram illustrating a flow feeder of a medical device at a first volume according to an example of the present disclosure.
Fig. 10 is a schematic diagram illustrating a flow feeder of a medical device at a second volume according to an example of the present disclosure.
Fig. 11 is a flowchart illustrating a control method of a medical device according to an example of the present disclosure.

### DETAILED DESCRIPTION

The technical solutions in the embodiments of the present disclosure will be clearly and completely described below in conjunction with the accompanying drawings in the embodiments of the present disclosure; obviously, the described embodiments are only a part of the embodiments of the present disclosure, rather than all the embodiments. Based on the embodiments of the present disclosure, all other embodiments filled by one of the ordinary skills in the art without involving any inventive efforts are within the scope of the present disclosure.

It needs to be noted that the terms "first", "second", "third", "fourth", and the like in the description and the claims of the present disclosure, as well as in the above-mentioned drawings, are used for distinguishing between different objects and not for describing a particular sequence. Furthermore, the terms "comprise" and "have", as well as any variations thereof, are intended to cover a non-exclusive inclusion. For example, a process, method, system, product, or device that comprises a list of steps or units is not limited to the listed steps or units, but may optionally further comprise steps or units not listed, or may optionally further comprise other steps or units inherent to such process, method, product, or device. In the following description, the same components are given the same symbols such that the repeated description is omitted. In addition, the drawings are merely schematic, and the proportions of the dimensions of the components relative to each other, or the shapes of the components, etc. may differ from reality.

The present disclosure relates to a medical device and a medical system for fluid conveyance. In the present disclosure, a medical device for conveying a fluid may be referred to simply as a medical device or a device. A patient may refer to a user of a medical device to which the present disclosure relates; the present disclosure is not intended to be limiting with respect to the terms of "user", "patients", or "patient", or the like, i.e., the meaning of the preceding words can sometimes be equivalent. Similarly, "fluid conveyance", "transfusion", "injected fluid", and the like are not intended to be limiting and can be construed in the same or similar sense unless otherwise specifically limited.

In addition, in the present disclosure, the fluid is not particularly limited and may be, for example, a medical solution injected through a medical device or a medical system to which the present disclosure relates; in some examples, such medical solutions can be dopamine, dobutamine, adrenaline, noradrenaline bitartrate, sodium nitroprusside, stilamin, propofol, insulin, glucagon-like peptide-1, etc. In addition, the medical device to which the present disclosure relates may also be used for regular, continuous, and precise drug delivery to a patient in conjunction with the actual situation of any disease.

One aspect of the present disclosure relates to a medical system for automatic drug delivery; the medical system may include any of the medical devices (described later) according to another aspect of the present disclosure.

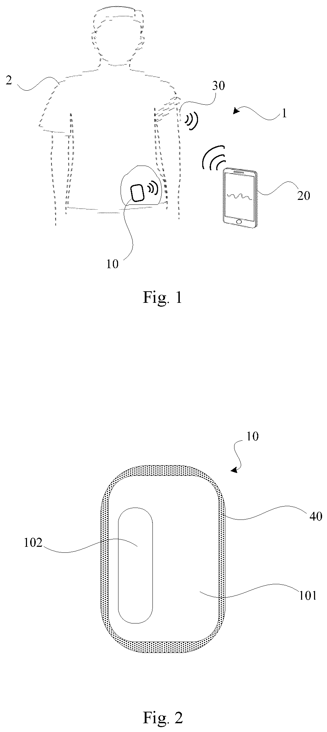

Fig. 1 is a schematic diagram illustrating an application scenario of an embodiment of a medical system 1 to which an example of the present disclosure relates.

As shown in Fig. 1, in some examples, a medical system 1 for automatic drug delivery may include any of the medical devices 10 (described later) according to another aspect of the present disclosure. In some examples, the medical system 1 may include devices such as a medical device 10, an external controller 20, and a sensing monitor 30. The medical system 1 for automatic drug delivery may hereinafter be simply referred to as the medical system 1 or the system 1. In some examples, the medical device may act on user 2 and may provide user 2 with transfusion therapy with quantitative control and high control accuracy.

In some examples, the external controller 20 may be a terminal apparatus such as a dedicated controller, a mobile phone, a personal computer, etc.; in some examples, the external controller 20 may also be a cloud apparatus or an Internet apparatus, for example, a server or a control terminal, etc. of an Internet hospital may be used as the external controller 20.

In some examples, the sensing monitor 30 may be a device used for monitoring a physiological parameters of a user, such as blood oxygen saturability, pulse rate, body temperature, height/weight, body composition, blood lipids, blood glucose, blood pressure, etc. This may not be limited herein.

In some examples, the sensing monitor 30 may be in wireless communication with the external controller 20. In some examples, wireless communication may include, but is not limited to, at least one of Bluetooth, Wifi, 3G/4G/5G, NFC, UWB, and Zig-Bee.

In some examples, the external controller 20 may be in wireless communication with the medical device 10. In some examples, wireless communication may include, but is not limited to, at least one of Bluetooth, Wifi, 3G/4G/5G, NFC, UWB, and Zig-Bee. In other examples, the external controller 20 may not be provided and the medical device 10 may have an internal controller or a control chip (not shown) that may be communicatively connected to the sensing monitor 30. In this case, the medical device 10 can directly respond to the monitoring data of the sensing monitor 30 and automatically provide the transfusion therapy to a patient in time, thereby improving the convenience and rapidity of the therapy.

In some examples, the sensing monitor 30 may obtain data of physiological parameters of the user and send the data to the external controller 20; the external controller 20 may control the medical device 10 to convey the fluid based on the data of the physiological parameters of the user.

In some examples, the sensing monitor 30 may be a blood glucose monitor by taking the treatment of diabetes as an example. The data of the physiological parameters of user 2 may be blood glucose data. The workflow of the medical system 1 may include: obtaining and sending real-time blood glucose data from the blood glucose monitor (i.e., the above-mentioned sensing monitor 30) of the user 2 to the external controller 20; and the external controller 20 sending a control signal to the medical device 10 20 based on the blood glucose data; the medical device 10 delivering fluid into the body of the user 2 based on the control signal.

Therefore, according to the present disclosure, it is able to provide a medical system for conveying a medical solution with high precision.

Another aspect of the present disclosure relates to a medical device for conveying a medical solution with high precision. In some examples, the medical device may be used to form any of the above-mentioned medical systems of the present disclosure with other devices or instruments, that is, the medical device to which the present disclosure relates may be one of the constituent elements of any of the above-mentioned medical systems.

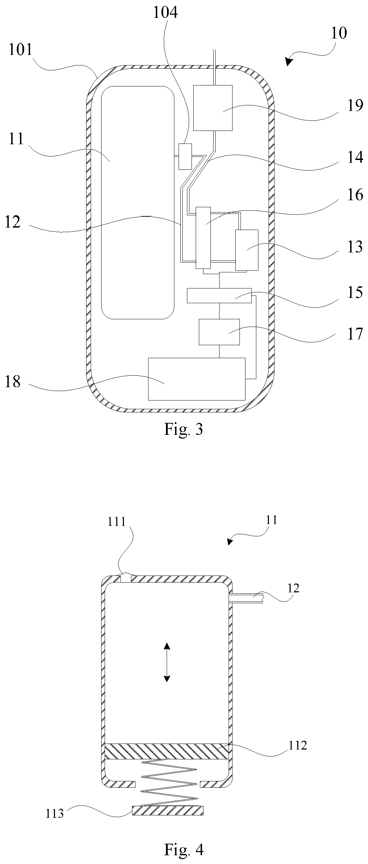

Fig. 2 is an overall schematic diagram showing a medical device 10 in a medical system 1 to which an example of Fig. 1 of the present disclosure relates. Fig. 3 is a schematic diagram showing the structure of a medical device 10 to which an example of the present disclosure relates.

As shown in Fig. 3, a medical device 10 according to the present disclosure may include: a reservoir 11, a first passage 12, a flow feeder 13, a driving mechanism 15, a second passage 14, and a flow-limiting valve 16.

In some examples, the reservoir 11 may be used to contain fluid, the first passage 12 may be in communication with the reservoir 11, the flow feeder 13 may be used to receive fluid from the reservoir 11 via the first passage 12 and provide the fluid, the driving mechanism 15 may be used to control the volume change of the flow feeder 13, the second passage 14 may be in communication with the flow feeder 13 and receive fluid from the flow feeder 13 and percutaneously access a body, and the flow-limiting valve 16 may be used in conjunction with the driving mechanism 15 and may be used to control the opening and closing of first passage 12 and the opening and closing of the second passage 14.

In some examples, the flow-limiting valve 16 may have a first state and a second state; when the flow-limiting valve 16 is in the first state, the driving mechanism 15 may maintain the flow feeder 13 at a first volume, and the flow feeder 13 may receive a predetermined volume of fluid from the reservoir 11 via the first passage 12; when the flow-limiting valve 16 is in the second state, the driving mechanism 15 may maintain the flow feeder 13 at a second volume, the flow feeder 13 may provide a predetermined volume of fluid via the second passage 14, the second volume may be smaller than the first volume, and the predetermined volume may be determined by a volume difference between the first volume and the second volume.

In this case, through storing the fluid in the reservoir 11 of the medical device 10, it is convenient for the patient to carry a predetermined dose of the fluid for injection in case of need so that the patient can receive treatment in time; by means of the cooperation between the flow feeder 13, the driving mechanism 15, and the flow-limiting valve 16, a predetermined volume of fluid is obtained from the reservoir 11 via the first passage 12, i.e., the difference value between the first volume and the second volume of the flow feeder 13, and the predetermined volume of fluid is infused into the body via the second passage 14, whereby it is able to obtain a predetermined volume (i.e., a unit quantity or a base quantity) of fluid for transfusion via the medical device 10 when the patient needs the injection of fluid so as to achieve the effect of digital quantity control, thereby improving the accuracy of the transfusion. In some examples, the predetermined volume of fluid may also be referred to as a unit quantity, a base quantity, or a digital quantity of fluid, and the predetermined volume of fluid may be determined by varying the first volume or the second volume of the flow feeder 13 according to different fluid types and treatment effects, for example, according to the scenario that diabetic patients need specific therapeutic effects, the quantity of insulin of a predetermined volume obtained each time may be set as 0.1mg (or ml), 0.5mg (or ml), 1mg (or ml), etc.

As shown in Fig. 3, in some examples, the medical device 10 may also include a control chip 17 used for responding to an external control signal from an external controller 20 (see Fig. 1), and the control chip 17 may send a control instruction to control the driving mechanism 15 based on the external control signal.

In some examples, control chip 17 may include a control module and a communication module. The control module (not shown) may be used to generate a control instruction based on the external control signal to control the driving mechanism 15, for example, start, pause, continue, or stop controlling. The communication module (not shown) may be used to do communication and data interaction with the external controller 20, for example, receiving an external control signal or sending the working state information, etc. of the current medical device 10.

As described above, the medical device 10 may infuse fluid into a patient based on a control signal. Specifically, the medical device 10 may generate a control instruction based on the control signal and control the driving mechanism 15 to drive the flow-limiting valve 16 and flow feeder 13 to obtain a predetermined volume of fluid and infuse the fluid into the patient. Therefore, it is able to achieve the effect of precise control of the digital quantity and improve the accuracy of the transfusion.

In some examples, the control chip 17 of the medical device 10 may be directly responsive to the data of a physiological parameter of a human body monitored by the sensing monitor 30 (see Fig. 1) and control the transfusion of fluid into the patient based on the data. In this case, the response time can be reduced compared to the aforementioned process of controlling the transfusion in response to the external control signal of the external controller 20 so that the patient can be more quickly assisted in getting treatment.

As shown in Fig. 3, in some examples, the medical device 10 may also include a battery module 18. The battery module 18 may be used to provide power to the driving mechanism 15 and the control chip 17. In some examples, the battery module 18 may also be used to provide power to other modules in the medical device 10 that require electric power. In some examples, the battery module 18 may be an energy module having an independent power source such as a lithium battery, a button battery, or the like. In other examples, the battery module 18 may be an energy storage module that is charged by an interface such as a USB or Type-C.

As shown in Fig. 3, in other examples, a medical device 10 for delivering fluids may include: a reservoir 11, a first passage 12, a flow feeder 13, a driving mechanism 15, a second passage 14, a flow-limiting valve 16, a needle assisting device 19, and a filter 104.

In some examples, a needle assisting device 19 may be used to insert the extension of the second passage 14 subcutaneously. Thereby, the medical device 10 can infuse fluids into the body through the second passageway 14.

In some examples, the subcutaneously inserted portion of the needle assisting device 19 may be a needle or a trocar, the needle assisting device 19 may advance the extension of the second passage 14 subcutaneously in a single pass and withdraw the needle or trocar leaving the extension of the second passage 14 subcutaneously. Thus, the medical device 10 can infuse fluids into the body through the second passageway 14 and can reduce the patient's pain from multiple needle sticks.

In some examples, the medical device 10 may not include the needle assisting device 19. In this case, the medical device 10 can be connected to the transfusion tube provided in the patient's body through the second passage 14and the transfusion can be accomplished, thereby enabling the patient who does not easily receive the drug carried in the application mode to perform the transfusion or facilitating the use in different scenarios.

In some examples, a filter 104 may be provided between the reservoir 11 and the first passage 12. The filter 104 may be configured to filter the fluid prior to the fluid provision of the reservoir 11 to the first passage 12. In this case, it is able to reduce a clogging situation caused by possible crystallization of the fluid (e.g., a drug such as insulin), thereby allowing the fluid to smoothly flow into the flow feeder 13 and improving the accuracy of fluid conveyance into the patient. In other examples, the medical device 10 may not be provided with a filter 104, for example, t the filter 104 may not be provided as the fluid is a medical fluid that does not easily crystallize.

In other examples, the medical device 10 for conveying fluids may also include an alarm device (not shown). The alarm device may be configured to detect the condition of the fluid in the reservoir 11. When the fluid storage amount is less, an alarm information can be sent, for example, a vibration or a sound, to prompt the patient to supply.

Refer to Figs. 2 or 3, in some examples, the medical device 10 may include a housing 101. The housing 101 may serve as a housing for the medical device 10 to protect the internal components of the medical device 10. In some examples, the housing 101 may be designed as shape of a flat prolonged block with an arc that can be adapted to the skin surface. In some examples, one surface of the housing 101 may be assembled with an application film 40 for attaching to the skin, i.e., the medical device 10 may be of the application type. In this case, the medical device 10 of the application type can be conveniently carried on by the patient and, in addition, can be used to treat patients by transfusion in time in the event of an emergency.

In some examples, the housing 101 may be provided with a transparent window 102 that visually displays the fluid volume of the reservoir 11 (see Fig. 2), thereby enabling the patient to view and replenish the fluid in a timely manner.

In other examples, the housing 101 of the medical device 10 may be provided as shape of a non-flat prolonged block (e.g., a sphere or a cube), that is, the housing 101 may be provided as a general block, and the medical device 10 may not be assembled with the application film 40. In some examples, a housing provided as a general block may be provided with a hanging structure or mechanism or a structure or mechanism easy for hand-holding. In this case, the medical device can be conveniently used for the treatment in the ward, or transfusion treatment can be conveniently performed by some users who do not easily receive the way of carrying a drug by application.

As described above, the outline configuration of the housing 101 of the medical device 10 may not be limited, in other words, the housing 101 of the medical device 10 may be an applied or an externally non-applied device. In other examples, the medical device 10 may be provided to be an implanted subcutaneous or intracorporeal device, and the housing 101 may be formed into a particular shape by using a material that is biocompatible, for example, a device that accommodates different body parts, such as an implantable antinociceptive pump, a hepatic vascular completely implantable drug pump, etc.

Fig. 4 is a schematic diagram showing the structure of a reservoir 11 of a medical device 10 to which an example of the present disclosure relates.

As shown in Fig. 4, in some examples, the volume of the reservoir 11 may be variable, and the pressure intensity of the fluid of the reservoir 11 may be maintained within a predetermined range when the reservoir 11 accommodates the fluid. The pressure intensity of the fluid of the reservoir 11 may be greater than the pressure intensity of the fluid of the flow feeder 13. In this case, the fluid is stored in the reservoir 11 and maintained within a predetermined pressure intensity range, enabling the fluid to more easily flow through the first passage 12 into the flow feeder 13, and serving to reduce fluid backflow or the occurrence of the blood entering the medical device 10 resulting in a contaminated fluid.

In some examples, the reservoir 11 may have a sealable supply port 111. In this case, the reservoir 11 can be periodically replenished with fluid through the supply port 111; in addition, the sealable supply port 111 can maintain the pressure intensity inside the reservoir 11 or reduce the situation of the air entrance or contamination caused by the external environment as the reservoir 11 is replenished with the fluid. In some examples, the location of the supply port 111 at the reservoir 11 may not be limited. In some examples, supply port 111 may cooperate with an external conduit to supply fluid to reservoir 11.

In some examples, one side of the reservoir 11 may be open and sealed by a first piston 112 and the other side of the reservoir 11 may be in communication with the first passage 12. In this case, it is able to push the fluid out of the first passage 12 through the piston and maintain a predetermined pressure intensity when the fluid is accommodated in the reservoir 11 so that it is able to reduce the backflow of the fluid or a situation of the blood flowing into the medical device 10 result in contaminating the fluid in the reservoir 11.

In some examples, the first piston 112 may be connected to a resilient component 113, the resilient component 113 may be maintained in a compressed state. In this case, the resilient component 113 maintained in the compressed state can drive the first piston 112 to displace so as to maintain the fluid contained in the reservoir 11 at a predetermined pressure intensity, thereby reducing the fluid backflow or a situation of blood backflowing to contaminate the fluid in the reservoir 11, etc.

In other examples, the reservoir 11 may also be a retractable container made of a resilient material capable of maintaining the pressure intensity within a predetermined range as accommodating the fluid. For example, silica gel, rubber, and the like. In this case, the above-mentioned effect can be achieved without providing the first piston 112 and the resilient component 113.

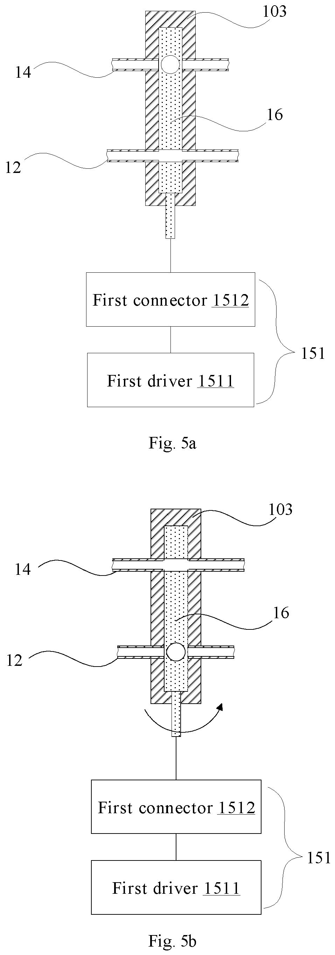

As mentioned above, the medical device 1 may include a flow-limiting valve 16. Fig. 5a is a cross-sectional diagram showing a flow-limiting valve 16 (in a first state) of a medical device 10 being connected to a first passage 12 and a second passage 14 according to an example of the present disclosure. Fig. 5b is a cross-sectional diagram showing a flow-limiting valve 16 (in a second state) of a medical device 10 being connected to a first passage 12 and a second passage 14 according to an example of the present disclosure.

Refer to Figs. 5a and 5b, in some examples, as the flow-limiting valve 16 is in the first state, the flow-limiting valve 16 may open the first passage 12 and close the second passage 14; as the flow-limiting valve 16 is in the second state, the flow-limiting valve 16 may close the first passage 12 and open the second passage 14. In this case, as the first passage 12 is opened and the second passage 14 is closed, the flow feeder 13 communicating with the first passage 12 can be driven to change the volume to a first volume by the driving mechanism 15, and a negative pressure can be formed in the flow feeder 13to obtain fluid from the reservoir 11 communicating with the first passage 12 via the first passage 12; as the first passage 12 is closed and the second passage 14 is opened, the volume of the flow feeder 13 communicating with the second passage 14 can be restored to its original volume, i.e., the second volume, and the fluid can be infused into the body via the second passage 14.

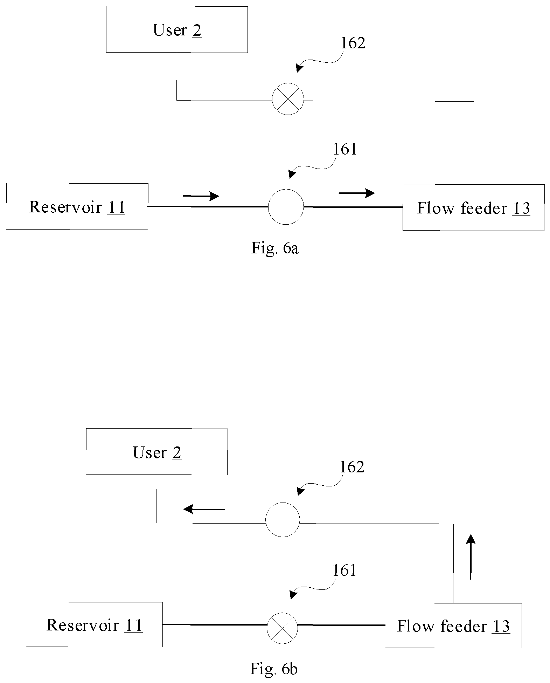

Fig. 6a is a schematic diagram illustrating the flow direction of the fluid in a medical device 10 as a flow-limiting valve 16 is in a first state and a flow feeder 13 is at a first volume according to an example of the present disclosure; Fig. 6b is a schematic diagram illustrating the flow direction of the fluid in a medical device 10 as a flow-limiting valve 16 is in a second state and a flow feeder 13 is at a second volume according to an example of the present disclosure.

As shown in Figs. 6a and 6b, in some examples, the flow-limiting valve 16 may include a first valve 161 controlling the opening and closing of the first passage 12 and a second valve 162 controlling the opening and closing of the second passage 14; as the first valve 161 closes the first passage 12, the second valve 162 opens the second passage 14, as the first valve 161 opens the first passage 12, the second valve 162 closes the second passage 14. In this case, as the first valve 161 opens the first passage 12 and the second valve 162 closes the second passage 14, the volume of the flow feeder 13 in communication with the first passage 12 can be driven by the driving mechanism 15 to change to the first volume so that negative pressure can be formed in to obtain fluids from the reservoir 11 in communication with the first passage 12 via the first passage 12; as the first valve 161 closes the first passage 12 and the second valve 162 opens the second passage 14, the volume of the flow feeder 13 in communication with the second passage 14 can be restored to the original volume, i.e., a second volume, and the fluid can be infused into the body via the second passage 14; thereby, through continuously interchanging of the first volume and the second volume, the flow feeder 13 can continuously obtain and infuse a predetermined volume (i.e. unit quantity or base quantity) of the fluid into the body.

In some examples, the first valve 161 and the second valve 162 may be provided as one of a stop-valve-shaped valve, a cock-shaped (or spherical) valve, a gate-shaped valve, a swing-shaped valve, a butterfly-shaped valve, and a slide valve-shaped valve.

In some examples, the first valve 161 and the second valve 162 may be controlled simultaneously, that is, driven by the identical power source (e.g., the first driving mechanism 151 below). In this case, the flow feeder 13 obtains the predetermined volume of the fluid by simultaneously controlling the first valve 161 to open and the second valve 162 to close, or controlling the first valve 161 to close and the second valve 162 to open; therefore, the accuracy in obtaining the predetermined volume of the fluid by the flow feeder 13 can be improved.

In other examples, the first valve 161 and the second valve 162 may be independently controlled, that is, driven by different power sources. In this case, the first valve 161 and the second valve 162, which are independently controlled, can also have the aforementioned effect of controlling the first valve 161 to open and the second valve 162 to close, or controlling the first valve 161 to close and the second valve 162 to open so that the flow feeder 13 obtains a predetermined volume of fluid, and the delay in fluid flow is calculated into the control by combining a mathematical calculation, therefore, the accuracy of obtaining a predetermined volume of fluid by the flow feeder 13 can also be improved.

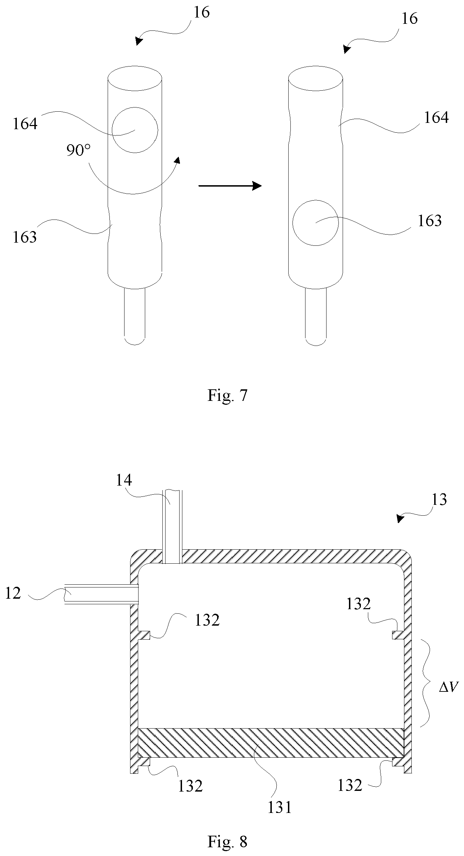

Fig. 7 is a schematic diagram illustrating the process of the rotation of a flow-limiting valve 16 of a medical device 10 from a first state to a second state according to an example of the present disclosure.

As mentioned above, in some examples, the medical device 10 may include a driving mechanism 15 (described later), the driving mechanism 15 includes a first driving mechanism 151 (see Figs. 5a and 5b).

In some examples, the flow-limiting valve 16 may be rotatably wrapped within the sealing material body 103 and may have an extension portion (not shown), the extension portion may be connected to and driven by the first driving mechanism 151 (see Figs. 5a and 5b). A sealing material body 103 may form a liquid flow cavity channel with the first passage 12 and the second passage 14 (see Figs. 5a and 5b).

In an exemplary embodiment of the present disclosure, the materials of the sealing material body 103, the first passage 12, and the second passage 14 may optionally be made of the same material, e.g., silica gel, or may be installed differently and designed to be made of different materials, e.g., the portion of the second passage 14 implanted into the patient may be made of a material with goods biocompatibility, e.g., polypropylene, siloxane, polyurethane, acrylic derivatives, polyhydroxy acids, etc.

As shown in Figs. 5a, 5b, and 7, in some examples, the flow-limiting valve 16 may be of a columnar body. In some examples, the flow-limiting valve 16 may have a first through hole 163 and a second through hole 164 (see Fig. 7). In some examples, the flow-limiting valve 16 may be columnar and have a first through hole 163 and a second through hole 164.

As shown in Figs. 5a and 5b, in some examples, the first driving mechanism 151 may include a first driver 1511, and a first connector 1512 driven by the first driver 1511 and connected to the flow-limiting valve 16. As the first driver 1511 drives the first connector 1512 to rotate the flow-limiting valve 16 to be in the first state, the first through hole 163 communicates with the first passage 12 and the second through hole 164 does not communicate with the second passage 14, as the first driver 1511 drives the first connector 1512 to rotate the flow-limiting valve 16 to be in the second state, the first through hole 163 does not communicate with the first passage 12 and the second through hole 164 communicates with the second passage 14.

In this case, as the flow-limiting valve 16 of the columnar body is driven by the first driving mechanism 151, the first through hole 163 can communicate with the first passage 12 and the second through hole 164 can not be in communication with the second passage 14, or the first through hole 163 can not be in communication with the first passage 12 and the second through hole 164 can communicate with the second passage 14; thereby, the flow-limiting valve 16 of the columnar body can control the fluid to flow or not flow via the first passage 12 or the second passage 14, while the inconvenience of designing multiple valves to control multiple passages can be reduced. In addition, the first driver 1511 can provide and transmit power to the flow-limiting valve 16 through the first connector 1512, so as to control the flow-limiting valve 16 to switch between the first state and the second state.

As shown in Fig. 7, in some examples, the first through hole 163 may have a first axis, the second through hole 164 may have a second axis, and the first axis and the second axis may be orthogonal. In this case, rotating the flow-limiting valve 16 of a columnar body shape is rotated, for example, 90 degrees clockwise or counterclockwise, the first through hole 163 can be in communication with the first passage 12 and the second through hole 164 can not be in communication with the second passage 14, or the first through hole 163 can be in communication with the first passage 12 and the second through hole 164 can not be in communication with the second passage 14. Thereby, the effect of controlling the opening and closing of the two passages at the same time by one power can be achieved.

In some examples, the first axis and the second axis may intersect spatially but not be parallel. In this case, the first through hole 163 having the first axis and the second through hole 164 having the second axis also enable the first through hole 163 to communicate with the first passage 12 and the second through hole 164 to not communicate with the second passage 14 or the first through hole 163 to not communicate with the first passage 12 and the second through hole 164 to communicate with the second passage 14 as the flow-limiting valve 16 is rotated to a predetermined angle. That is, two states that the first through hole 163 and the first passage 12 are in communication and the second through hole 164 and the second passage 14 are not n communication, or the first through hole 163 and the first passage 12 are not n communication and the second through hole 164 and the second passage 14 are n communication are satisfied.

In some examples, the rotation of the flow-limiting valve 16 may be unidirectional or reciprocating. Preferably, the embodiment the present disclosure employs the reciprocating rotation.

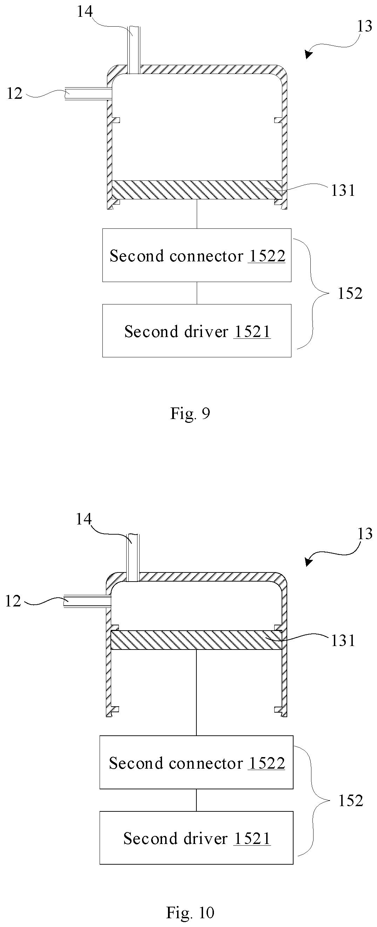

Fig. 8 is a schematic structural diagram showing a flow feeder 13 of a medical device 10 according to an example of the present disclosure; Fig. 9 is a schematic diagram illustrating a flow feeder 13 of a medical device 10 at a first volume according to an example of the present disclosure; Fig. 10 is a schematic diagram illustrating a flow feeder 13 of a medical device 10 at a second volume according to an example of the present disclosure.

As described above, in some examples, the flow-limiting valve 16 may have a first state and a second state, as the flow-limiting valve 16 is in the first state, the driving mechanism 15 may maintain the flow feeder 13 at the first volume (see Fig. 9), and the flow feeder 13 may receive a predetermined volume of the fluid from the reservoir 11 via the first passage 12; as the flow-limiting valve 16 is in the second state, the driving mechanism 15 may maintain the flow feeder 13 at the second volume (see Fig. 10), the flow feeder 13 may provide a predetermined volume of fluid ΔV via the second passage 14, the second volume may be smaller than the first volume, and the predetermined volume Δ*V* may be equal to the first volume minus the second volume (see Fig. 8).

As shown in Figs. 8, 9, and 10, in some examples, one side of the flow feeder 13 may be open and sealed by the second piston 131, and the other side of the flow feeder 13 may communicate with the second passage 14. In this case, the volume of the flow feeder 13 can be changed by the displacement of the second piston 131 and negative pressure can be formed at the first volume and a predetermined volume of the fluid can be obtained, and positive pressure may be recovered at the second volume and the fluid can be infused into the body through the second passage 14.

In some examples, the driving mechanism 15 may include a second driving mechanism 152, and the second piston 131 may be connected to the second connector 1522 and driven by the second driver 1521. In this case, the second driver 1521 provides and transmits power to the second piston 131 via the second connector 1522; thereby, the volume of the flow feeder 13 can be changed by the displacement of the second piston 131.

As shown in Fig. 8, in some examples, in order to ensure the accuracy of the volume change of the flow feeder 13, multiple limiting protrusions 132 may also be provided in the flow feeder 13. In this case, as the second piston 131 reciprocates in the flow feeder 13 to change the volume of the flow feeder 13, the limiting protrusions 132 can limit the second piston 131 to improve the accuracy of the volume change of the flow feeder 13.

In other examples, the flow feeder 13 may also be a retractable container formed of a resilient material, capable of expanding to a predetermined degree and creating a negative pressure when the filling of the fluid is required, and capable of retracting to the original shape to expel the fluid when the fluid is required to be expelled. In this case, the provision of the second piston 131 and the second connector 1522 can be reduced.

As the previously described driving mechanism 15, in some examples, the driving mechanism 15 may include the above-described first driving mechanism 151 and the above-described second driving mechanism 152. In some examples, the first driving mechanism 151 may control the flow-limiting valve 16 to open and close the first passage 12 or the second passage 14 (see Figs. 5a and 5b, or Figs. 6a and 6b), and the second driving mechanism 152 may control the volume change of the flow feeder 13 to switch the volume of the flow feeder 13 between the first volume and the second volume (see Figs. 9 and 10).

In this case, through the cooperation of the first driving mechanism 151 and the second driving mechanism 152, the first driving mechanism 151 drives the flow-limiting valve 16 to be in the first state, that is, as the first valve 161 opens the first passage 12 and the second valve 162 closes the second passage 14, the second driving mechanism 152 can control the volume of the flow feeder 13 to be maintained at the first volume; thereby, a negative pressure can be formed in the flow feeder 13and fluids can be obtained from the reservoir 11 via the first passage 12; as the fluid fills the first volume and the first driving mechanism 151 drives the flow-limiting valve 16 to be in the second state, that is, as the first valve 161 closes the first passage 12 and the second valve 162 opens the second passage 14, the second driving mechanism 152 can control the volume of the flow feeder 13 to be maintained at the second volume, thereby, the flow feeder 13 can infuse a predetermined volume of the fluid into the body.

In some examples, the first driving mechanism 151 and the second driving mechanism 152 may be controlled simultaneously, that is, as the first driving mechanism 151 controls the flow-limiting valve 16 to open and close the first passage 12 or the second passage 14, the second driving mechanism 152 simultaneously controls the volume change of the flow feeder 13 to switch the volume of the flow feeder 13 between the first volume and the second volume. In this case, the simultaneous control can reduce the time of the transfusion of the fluid into the body and reduce the number of sequential inputs of a control instruction, thereby enabling the simplification of a control program of a control chip 17 and enabling the speeding up of the treatment of a patient.

In some examples, the first driving mechanism 151 and the second driving mechanism 152 may be controlled in time delay manner, that is, as the first driving mechanism 151 controls the flow-limiting valve 16 to open and close the first passage 12 or the second passage 14, the second driving mechanism 152, following the control of the flow-limiting valve 16 by the first driving mechanism 151, controls the volume change of the flow feeder 13 to switch the volume of the flow feeder 13 between the first volume and the second volume. In this case, the time delayed control can improve the accuracy of the control and reduce the situations of poor treatment effect caused by wrong control sequences.

As shown in Figs. 5a or 6a, in some examples, when the first driving mechanism 151 enables the flow-limiting valve 16 to be at the first state, the flow-limiting valve 16 may open the first passage 12 and close the second passage 14, and the second driving mechanism 152 may control the flow feeder 13 to maintain the first volume and receive a predetermined volume of fluid from the reservoir 11 via the first passage 12. As shown in Figs. 5b or 6b, in some examples, when the first driving mechanism 151 enables the flow-limiting valve 16 to be at the second state, the flow-limiting valve 16 may close the first passage 12 and open the second passage 14, and the second driving mechanism 152 may control the flow feeder 13 to maintain the second volume and provide a predetermined volume of fluid via the second passage 14. In this case, the flow-limiting valve 16 is controlled to switch between the first state and the second state by the first driving mechanism 151, and the flow feeder 13 is controlled to switch between the first volume and the second volume by cooperating with the second driving mechanism 152, thereby, a predetermined volume of fluid can be continuously obtained and infused into the body, achieving quantitative or digital quantity control and improving the control accuracy of the transfusion.

As shown in Figs. 5a or 5b, in some examples, the second driving mechanism 152 may include a second driver 1521, and a second connector 1522 driven by the second driver 1521 and connected to the flow feeder 13.

In some examples, the fluid flows from the reservoir 11 to the flow feeder 13 via the first passage 12 if the second driver 1521 drives the second connector 1522 to change the volume of the flow feeder 13 to maintain the first volume, and fluid flows from the flow feeder 13 to the body via the second passage 14 if the second driver 1521 drives the second connector 1522 to change the volume of the flow feeder 13 to maintain the second volume. In this case, the second driver 1521 provides and transmits power to the flow feeder 13 via the second connector 1522 to change the volume of the flow feeder 13, thereby predetermined volume of fluid can be obtained from the reservoir 11 and infused into the body in cooperation with the first driving mechanism 151 and the flow-limiting valve 16.

In some examples, the first driver 1511 or the second driver 1521 may be one of a shape memory alloy, a piezoelectric motor, and a servo motor. The first connector 1512 may be one of a torsion spring and a gear. The second connector 1522 may be one of a spring and a connecting rod. In this case, the shape memory alloy is heated to a predetermined range by powering on so as to obtain the power generated by the deformation of the shape memory alloy so that it is able to drive the first connector 1512 and the flow-limiting valve 16, when the heating is stopped, the first connector 1512 and the flow-limiting valve 16 can be driven reversely when the power is generated by the deformation recovery of the shape memory alloy, which has the advantages of power saving, accurate control and fast response. The first connector 1512 and the flow-limiting valve 16 are driven by a piezoelectric motor or a servo motor, having the advantages of accurate control and fast response; in addition, the first connector 1512 constituted by a torsion spring or a gear can rotate and revolve the flow-limiting valve 16 under power drive, thereby opening and closing the first passage 12 or the second passage 14; in addition, the second connector 1522 constituted by a spring or a push rod can reciprocally displace the second piston 131 under power drive, thereby changing the volume of the flow feeder 13.

In order to better embody the functional effects of the medical device 10 to which the present disclosure relates, the present disclosure may also provide a method for controlling a medical device 10 for fluid conveyance. Hereinafter, it may be simply referred to as a control method or a method.

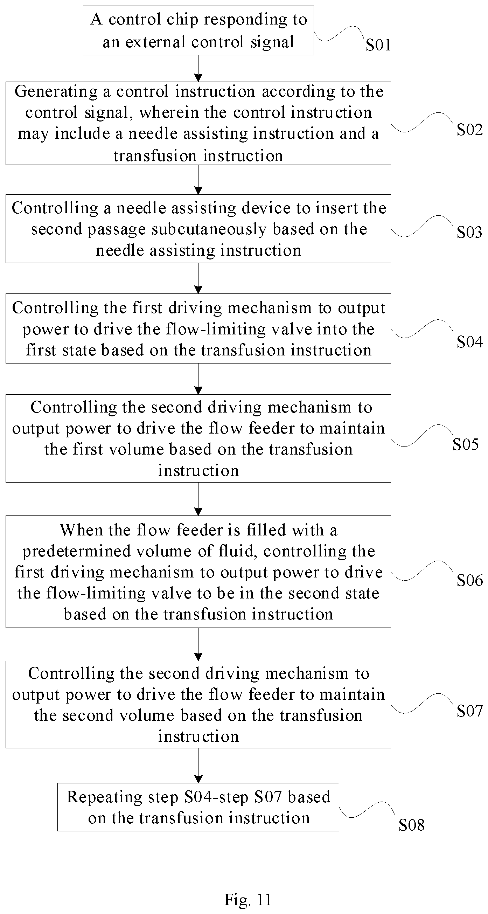

Fig. 11 is a flowchart illustrating a control method of a medical device 10 according to an example of the present disclosure.

In some examples, as shown in Fig. 11, the control method may include:
step S01: a control chip 17 responding to an external control signal.
Step S02: generating a control instruction according to the control signal, wherein the control instruction may include a needle assisting instruction and a transfusion instruction, the needle assisting instruction is used for controlling the needle assisting device 19 to insert the second passage 14 subcutaneously prior to delivering the fluid and the transfusion instruction is used for controlling the driving mechanism 15.
Step S03: controlling the needle assisting device 19 to insert the second passage 14 subcutaneously based on the needle assisting instruction.
Step S04: based on the transfusion instruction, controlling the first driving mechanism 151 to output power to drive the flow-limiting valve 16 into a first state, that is, the first passage 12 is opened and the second passage 14 is closed.
Step S05: based on the transfusion instruction, controlling the second driving mechanism 152 to output power to drive the flow feeder 13 to maintain the first volume.
Step S06: when the flow feeder 13 is filled with a predetermined volume of fluid, based on the transfusion instruction, controlling the first driving mechanism 151 to output power to drive the flow-limiting valve 16 into the second state, that is, the first passage 12 is closed and the second passage 14 is opened.
Step S07: based on the transfusion instruction, controlling the second driving mechanism 152 to output power to drive the flow feeder 13 to maintain a second volume, thereby a predetermined volume of the fluid is infused into the body through the second passage 14, wherein the second volume is smaller than the first volume, the predetermined volume is equal to the first volume minus the second volume.
Step S08: repeating step S04 to step S07 based on the transfusion instruction, thereby continuously providing a quantitative medical solution to the patient according to the required amount of medical solution and improving the therapeutic effect.

In some examples, steps S04 and S05 may be performed simultaneously, and steps S06 and S07 may be performed simultaneously.

In some examples, in step S04, the first driving mechanism 151 may output power reversely to drive the flow-limiting valve 16 in the second state, that is, the first passage 12 is closed and the second passage 14 is opened. In other words, the first driving mechanism 151 may drive the flow-limiting valve 16 to reciprocate so that the first passage 12 is switched between being opened and closed and the second passage 14 is switched between being closed and opened, that is, after step S04 is completed, step S06 may be completed by reciprocating motion.

In some examples, in step S05, the second driving mechanism 152 may output power reversely to maintain the flow feeder 13 at the second volume. In other words, the second driving mechanism 152 may drive the second piston 131 to reciprocate so that the volume of the flow feeder 13 is switched between the first volume and the second volume, that is, after step S05 is completed, step S07 may be completed by reciprocating motion.

According to another aspect of the present disclosure, a medical device 10 for delivering fluids with high precision can be provided. Wherein, the medical device controls the delivery of the fluid by a digital quantity (i.e., a predetermined volume of fluid) so that it is featured with high precision of fluid conveyance in comparison with the prior art.

According to the above two aspects of the present disclosure, it is able to provide a medical device and a medical system for conveying fluids with high precision.

Although the present disclosure has been specifically described above with reference to the accompanying drawings and examples, it will be understood that the above description does not limit the disclosure in any form. Those skilled in the art can make modifications and variations to the disclosure as required without departing from the true spirit and scope of the disclosure, and these modifications and variations fall within the scope of the disclosure.

## Claims

1. A medical device for fluid conveyance, **characterized by** including:
a reservoir for containing a fluid;
a first passage in communication with the reservoir;
a flow feeder for receiving the fluid from the reservoir via the first passage and providing the fluid;
a driving mechanism for controlling a volume change of the flow feeder;
a second passage, in communication with the flow feeder, receiving the fluid from the flow feeder and percutaneously accessing a body; and
a flow-limiting valve for cooperating with the driving mechanism and controlling an opening and closing of the first passage and the opening and closing of the second passage;
wherein the flow-limiting valve has a first state and a second state, the driving mechanism maintains the flow feeder at a first volume when the flow-limiting valve is in the first state, and the flow feeder receives a predetermined volume of fluid from the reservoir via the first passage; the driving mechanism maintains the flow feeder at a second volume when the flow-limiting valve is in the second state, and the flow feeder provides the predetermined volume of fluid via the second passage, the second volume is smaller than the first volume and the predetermined volume is determined by a volume difference between the first volume and the second volume.

2. The medical device according to claim 1, **characterized in that**
when the flow-limiting valve is in the first state, the flow-limiting valve opens the first passage and closes the second passage;
when the flow-limiting valve is in the second state, the flow-limiting valve closes the first passage and opens the second passage.

3. The medical device according to claim 1, **characterized in that**
a volume of the reservoir is variable, and when the reservoir contains the fluid, a pressure intensity of the fluid of the reservoir is maintained within a predetermined range, with the pressure intensity of the fluid of the reservoir being greater than the pressure intensity of the fluid of the flow feeder.

4. The medical device according to claim 1 or 3, **characterized in that**
the reservoir has a sealable supply port.

5. The medical device according to claim 1 or 2, **characterized in that**
the flow-limiting valve includes a first valve controlling the opening and closing of the first passage and a second valve controlling the opening and closing of the second passage, wherein the second valve opens the second passage if the first valve closes the first passage, and the second valve closes the second passage if the first valve opens the first passage.

6. The medical device according to claim 1, **characterized in that**
the driving mechanism includes a first driving mechanism and a second driving mechanism, wherein the first driving mechanism controls the flow-limiting valve to open and close the first passage or the second passage, and the second driving mechanism controls the volume change of the flow feeder to switch the volume of the flow feeder between the first volume and the second volume.

7. The medical device according to claim 6, **characterized in that**
when the first driving mechanism keeps the flow-limiting valve in the first state, the flow-limiting valve opens the first passage and closes the second passage, and the second driving mechanism controls the flow feeder to maintain the first volume and receive the predetermined volume of fluid from the reservoir via the first passage;
when the first driving mechanism keeps the flow-limiting valve in the second state, the flow-limiting valve closes the first passage and opens the second passage, and the second driving mechanism controls the flow feeder to maintain the second volume and provides the predetermined volume of fluid via the second passage.

8. The medical device according to claim 6, **characterized in that**
the flow-limiting valve is of a columnar body and has a first through hole and a second through hole;
the first driving mechanism includes a first driver, and a first connector driven by the first driver and connected to the flow-limiting valve; if the first driver drives the first connector so as to rotate the flow-limiting valve to be in the first state, the first through hole is in communication with the first passage, and the second through hole is not in communication with the second passage; if the first driver drives the first connector so as to rotate the flow-limiting valve to be in the second state, the first through hole is not in communication with the first passage, and the second through hole is in communication with the second passage.

9. The medical device according to claim 8, **characterized in that**
the first through hole has a first axis and the second through hole has a second axis, the first axis being orthogonal to the second axis.

10. The medical device according to claim 1 or 3, **characterized in that**
one side of the reservoir is open and sealed by a first piston, and the other side of the reservoir is in communication with the first passage.

11. The medical device according to claim 10, **characterized in that**
the first piston is connected to a resilient component, the resilient component being maintained in a compressed state.

12. The medical device according to claim 6, **characterized in that**
the second driving mechanism includes a second driver, and a second connector driven by the second driver and connected to the flow feeder; if the second driver drives the second connector to change the volume of the flow feeder to maintain the first volume, the fluid flows from the reservoir to the flow feeder via the first passage, and if the second driver drives the second connector to change the volume of the flow feeder to maintain the second volume, the fluid flows from the flow feeder to the body via the second passage.

13. The medical device according to claim 12, **characterized in that**
one side of the flow feeder is open and sealed by a second piston, and the other side of the flow feeder is in communication with the second passage.

14. The medical device according to claim 13, **characterized in that**
the second piston is connected to the second connector and driven by the second driver.

15. The medical device according to claim 8 or 12, **characterized in that**
the first driver or the second driver is one of a shape memory alloy, a piezoelectric motor, and a servo motor, the first connector is one of a torsion spring and a gear, and the second connector is one of a spring and a connecting rod.

16. A medical system, **characterized by**
including the medical device of any one of claims 1 to 15.

17. The medical system according to claim 16, **characterized by**
further including an external controller that controls the medical device and a sensing monitor communicatively connected to the external controller, wherein the sensing monitor obtains data of a physiological parameters of a user and sends the data of the physiological parameters of the user to the external controller, the external controller controlling the medical device to deliver fluids based on the data of the physiological parameters of the user.
